# EUROPEAN PATENT APPLICATION

(11) **EP 2 548 517 A1**
(43) Date of publication of application: **23.01.2013**
(21) Application number: 11756091.2
(22) Date of filing: 03.03.2011
(51) Int. Cl.: A61B 17/00, A61B 1/00, A61B 18/00, A61B 18/12, A61B 18/18, A61B 18/20, A61M 25/00, A61M 31/00

(54) **CATHETER FOR ENDOSCOPE**

(30) Priority: 07.09.2010 JP 2010199913; 19.03.2010 JP 2010064882
(71) Applicant: Yamashina Seiki Co., Ltd., Ritto-shi Shiga 520-3001 (JP); Osaka University, Suita-shi Osaka 565-0871 (JP)
(72) Inventor: NAKAJIMA, Kiyokazu, Suita-shi Osaka 565-0871 (JP); HOSAKA, Makoto, Ritto-shi Shiga 520-3001 (JP); IDETA, Tomoya, Ritto-shi Shiga 520-3001 (JP)
(74) Representative: MacLean, Martin Robert
(86) International application number: PCT/JP2011/054971
(87) International publication number: WO 2011/114902

(57) **Abstract**

The catheter (1, 11) for an endoscope of the present invention comprises an openings (2, 12) in the vicinity of the tip at a distal end thereof, wherein the opening has a diameter smaller than an inner diameter of the catheter, and a plurality of said openings are provided. The catheter for an endoscope of the present invention can have multiple functions such as irrigation, suction, incision, cauterization, hemostasis by coagulation, and smoke evacuation.

## Description

### Technical Field

The present invention relates to a catheter for an endoscope, having multiple functions such as irrigation, suction, incision, cauterization, hemostasis by coagulation, and smoke evacuation.

### Background Art

In clinical departments, direct observations and procedures in the body cavity are performed in order to diagnose various disease states, see the effects of treatment, determine treatment policies, and the like. Conventionally, these procedures have been performed with a highly invasive approach such as a laparotomy or a thoracotomy. However, with the recent widened use of endoscopic surgical instruments, these procedures are performed using an endoscope such as a laparoscopy or a thoracoscope in which the size of incised wounds is smaller than that in conventional cases.

For example, at the time of incision or cauterization during endoscopic surgeries, usually, electrode forceps are used. In this case, it is desirable to irrigate the mucosal surface targeted for the procedure before the incision or cauterization, and to irrigate and remove cauterized pieces that have adhered to the electrode forceps or the peripheral tissue during the incision or cauterization. However, devices used via an instrument channel of a current endoscope have poor procedure performance for unexpected bleeding or smoke generation during incision or cauterization. For example, for unexpected bleeding, a face of an endoscope lens is immersed in blood, and the blood is sucked from an instrument channel. With this method, there is a problem in that, since the endoscope lens is immersed in blood, the field of view of the endoscope is lost during the suction, which makes it difficult to achieve rapid hemostasis. The field of view of the endoscope is impaired also by smoke generated due to incision or cauterization, but current devices cannot efficiently evacuate the smoke.

A currently used catheter for an endoscope is formed by merely cutting a tube. If such a catheter is used as it is, for example, mucosa, tissue, organs that are not to be sucked are sucked together with the target. Furthermore, such a device has only one suction opening, and, therefore, if this opening is blocked due to miss-suction of adjacent tissue/organ, subsequent suction operations cannot be performed. Moreover, in most cases, such a device is a single-function device. Accordingly, instruments necessary for procedures and operations such as irrigation or suction have to be switched each time through an instrument channel, causing problems such as the operation becoming complicated, the burden on a surgeon increasing, the cost of the instruments increasing, and the time for procedures and operations lengthening.

Research has been conducted to provide a device for an endoscope with various functions. For example, Patent Document 1 discloses an apparatus including an electrically insulating sheath that can be inserted into or removed from an instrument channel of an endoscope and a high-frequency electrode that is provided inside the sheath and that is protruded forward from and withdrawn into the tip of the sheath through an operation on the operator's side of the sheath, wherein a space surrounding the high-frequency electrode is used as a water supply path, and water is ejected from the tip of the sheath. Patent Document 2 discloses an improved version of the apparatus that ejects water from the tip of the sheath according to Patent Document 1. Patent Document 3 discloses a hemostatic instrument including a high-frequency electrode that is provided with a water supply mechanism for irrigation. However, these apparatuses have to use another device in order to perform suction.

### Prior Art Documents

### Patent Documents

Patent Document 1: Japanese Laid-Open Patent Publication No. 6-292685
Patent Document 2: Japanese Laid-Open Patent Publication No. 2006-187474
Patent Document 3: Japanese Laid-Open Patent Publication No. 2002-125981

### Summary of Invention

### Problems to be Solved by the Invention

It is an object of the present invention to provide a device for an endoscope, capable of having multiple functions such as irrigation, suction, incision, cauterization, hemostasis by coagulation, and smoke evacuation.

### Means for Solving the Problems

The present invention provides a catheter for an endoscope, comprising an opening in the vicinity of the tip at a distal end thereof, wherein the opening has a diameter smaller than an inner diameter of the catheter, and a plurality of said openings are provided.

In one embodiment, the endoscope is a flexible endoscope.

In one embodiment, the vicinity of the tip has a cylindrical shape, and the number of said openings along an outer circumference of the cylindrical shape is 3 to 12 per circumference.

In one embodiment, the catheter further comprising an energy element in the vicinity of the tip.

In one embodiment, the energy element generates at least one selected from the group consisting of a high-frequency current, a radio wave, a microwave, an ultrasonic wave, and a laser beam.

### Effects of Invention

The catheter for an endoscope of the present invention can irrigate the lumen surface targeted for procedures, suck the blood, and irrigate and remove cauterized pieces that have adhered to the electrode forceps or the peripheral tissue during incision or cauterization, via a plurality of openings that are arranged in the vicinity of the tip at the distal end of the catheter and that each have a diameter smaller than an inner diameter of the catheter. Furthermore, the catheter for an endoscope of the present invention can have not only the above-described functions but also other functions such as incision, cauterization, hemostasis by coagulation, and smoke evacuation. In this manner, the catheter for an endoscope of the present invention can have multiple functions such as irrigation, suction, incision, cauterization, hemostasis by coagulation, and smoke evacuation.

### Brief Description of Drawings

[Fig. 1] Fig.1 is a schematic diagram showing a partial perspective view of the first embodiment of the catheter for an endoscope of the present invention.
[Fig. 2] Fig.2 is a schematic diagram showing a partial perspective view of the second embodiment of the catheter for an endoscope of the present invention.
[Fig. 3] Fig.3 is a graph showing a result comparing flow rate performance between the catheter for an endoscope of the present invention and a conventional endoscope.
[Fig. 4] Fig. 4 is a graph showing relationship between pressure and flow rate of the catheter for an endoscope of the present invention.

### Mode for Carrying Out the Invention

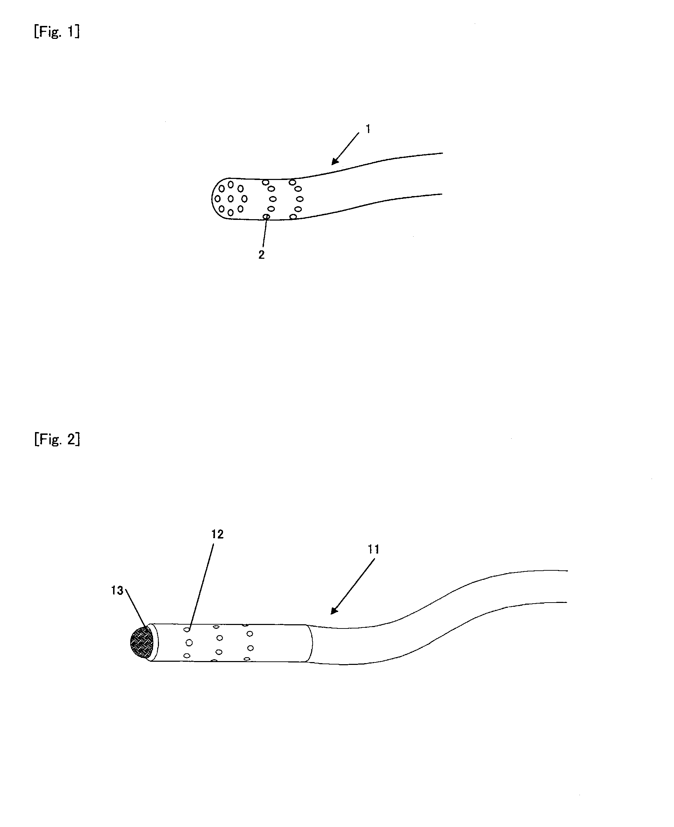

A catheter 1 of a first embodiment of the present invention shown in Fig. 1 is provided with an opening 2 in the vicinity of the tip at a distal end thereof, the opening having a diameter smaller than an inner diameter of the catheter. A plurality of such openings are provided.

In this specification, the "catheter" refers to a medical instrument that is a hollow and soft tube used in the medical field, and that is inserted into the lumen such as the digestive tract or the ureter, the peritoneal cavity, or the like, and used for suction of an irrigation fluid or a body fluid, injection of a liquid medicine or a contrast medium, or the like.

There is no particular limitation on the object hollow organ, but examples thereof include the bronchi, the esophagus, the stomach, the small intestine, the colon and rectum, the vagina, the bladder, the thoracic cavity, and the peritoneal cavity.

The catheter of the present invention includes a distal end portion that has a distal end, a proximal end portion that has a proximal end, and a guide tube portion that intervenes between the distal end portion and the proximal end portion. There portions may be integrally formed or may be connected to each other as appropriate using joints.

In this specification, the "endoscope" refers to an endoscope for medical use. Examples of the endoscope include a flexible endoscope in which a portion inserted into the body can be curved and a rigid endoscope in which that portion cannot be curved. The rigid endoscope has a simple structure in which lenses are arranged at both ends of the tube. Examples of the rigid endoscope include a cystoscope, a thoracoscope, and a laparoscopy. The flexible endoscope is flexible, and an observation optical system embedded therein may be of a glass fiber type or of a CCD type. Furthermore, typically, an illumination optical system thereof is provided with a light source on the proximal end side outside the body, and guides light through optical fibers inside the guide tube portion and irradiates the light from the distal end side. Also, the illumination optical system may be of a type where an LED is embedded in the distal end of the endoscope. Examples of the flexible endoscope include a bronchoscope, an esophagogastroduodenoscope (gastroscope, etc.), an enteroscope, and a colonoscope. Such an endoscope typically has an instrument channel (sub lumen) in addition to these optical systems. It is possible to perform, via this channel, injection and suction of gas or liquid, and procedures (grip, cutting, puncture, etc.) using a dedicated device, for example. Furthermore, the orientation of the distal end of the endoscope can be freely changed through an operation on the proximal end side. An endoscope of an appropriate size is selected according to the object lumen.

The catheter of the present invention has an outer diameter that allows the catheter to be inserted into the instrument channel of such an endoscope. The inner diameter of the instrument channel is usually about 1 to 4 mm. Accordingly, the outer diameter of the catheter of the present invention may be smaller than 1 to 4 mm.

The length of the catheter of the present invention may be any length as long as, when the catheter is inserted into the instrument channel of the endoscope, the distal end of the catheter is sufficiently protruded (or exposed) from the instrument channel into the lumen to perform procedures and operations. Usually, the length may be similar to that of a wire or the like extended to a treatment instrument for the endoscope.

There is no particular limitation on the wall thickness of the catheter of the present invention as along as it provides a flexibility and a strength that allow the distal end of the catheter to be sent via the instrument channel to the tip of the endoscope and protruded (or exposed) into the lumen. The wall thickness is preferably 0.3 mm or less.

There is no particular limitation on the material of the catheter of the present invention as long as it provides a flexibility, a strength, a low frictional performance, and, if necessary, an insulating performance that allow the distal end of the catheter to be sent via the instrument channel to the tip of the endoscope and protruded (or exposed) into the lumen. Examples of such a material include flexible resins such as polyvinyl chloride, polyethylene, polyester, polyurethane, polyamide, silicone resin, PTFE, PFA, polypropylene, nylon, polyether ether ketone (PEEK), and POM. These materials may be used alone or in a combination with another material.

There is no particular limitation on the cross-sectional shape of the catheter of the present invention as long as the catheter is hollow, but the catheter is preferably in the shape of a hollow concentric cylinder.

There is no particular limitation on the shape of the tip at the distal end of the catheter of the present invention, but it may be a flat shape or may be a round protruding shape.

There is no particular limitation on the shape of the vicinity of the tip at the distal end of the catheter of the present invention, but it is preferably a cylindrical shape.

The vicinity of the tip refers to a distal end portion that is within 50 mm, preferably 10 mm, from the tip at the distal end.

The catheter of the present invention is provided with an opening in the vicinity of the tip at the distal end thereof, the opening having a diameter smaller than an inner diameter of the catheter. A plurality of such openings are provided.

There is no particular limitation on the shape of the opening, but it is preferably a circular shape or an elliptical shape.

The diameter of the opening is preferably 0.2 mm or more, more preferably 0.3 mm or more, and is preferably no wider than 0.6 mm, more preferably no wider than 0.5 mm. The diameter of the opening is preferably small so that tissue isn't taken in at the time of suction and an irrigation fluid can be vigorously ejected to a wide range of the lumen surface at the time of irrigation.

There is no particular limitation on the number of openings, but it is preferably 15 or more, more preferably 20 or more, and is preferably no more than 50, more preferably no more than 30.

If the vicinity of the tip at the distal end has a cylindrical shape, the number of openings along the outer circumference of the cylindrical shape is preferably 3 to 12 per circumference, more preferably 8 per circumference.

The openings may be arranged over several circumferences, preferably over 2 to 4 circumferences.

There is no particular limitation on the arrangement of openings. If multiple openings are arranged, the openings may be arranged at constant intervals or may be arranged at varying intervals. For example, the openings may be arranged in a grid-like or helical pattern, or may be arranged randomly.

The openings are preferably arranged such that the same number of openings per circumference are linearly aligned in the longitudinal direction. Alternatively, the openings are arranged such that the same number of openings per circumference are sequentially arranged between adjacent circumferences by a predetermined angle along the outer circumference. With the arrangement in which the same number of openings per circumference are linearly aligned in the longitudinal direction, ejection can be performed in the same direction, and, therefore, for example, this arrangement can provide an intense irrigation force. With the arrangement in which the same number of openings per circumference are sequentially arranged between adjacent circumferences by a predetermined angle along the outer circumference, ejection can be performed in different directions, and, therefore, for example, this arrangement is useful to uniformly spray an agent. For example, with the arrangement in which 8 openings per circumference are arranged along the outer circumference for 3 circumferences, and the openings are sequentially arranged by 15° along the outer circumference, ejection can be performed in 24 directions.

If necessary, the proximal end of the catheter of the present invention is connected via ejection means or suction means respectively to an irrigation fluid supply source or a waste fluid receiver.

In the catheter of the present invention, the ejection means on the proximal end side causes an irrigation fluid to be sent from the irrigation fluid supply source into the guide tube portion, transmitted via the openings in the vicinity of the tip at the distal end, and ejected to the lumen surface. The suction means on the proximal end side causes a body fluid such as the blood on the lumen surface, a waste fluid after irrigating the lumen surface, and smoke generated due to incision or cauterization using a high-frequency electrode to be sucked from the openings in the vicinity of the tip at the distal end, transmitted via the guide tube portion, and sent to the waste fluid receiver on the proximal end side. The ejection means and the suction means are switched as appropriate through an operation on the proximal end side, and can be operated while monitoring the lumen surface using the endoscope.

There is no particular limitation on the flow rate performance of the catheter. The ejection flow rate performance is preferably 150 to 600 mL/min, more preferably 250 to 450 mL/min. The suction flow rate performance is preferably 100 to 400 mL/min, more preferably 200 to 300 mL/min.

A catheter 11 of a second embodiment of the present invention shown in Fig. 2 is provided with an opening 12 in the vicinity of the tip at a distal end thereof, and further provided with an energy element 13.

The energy element generates, for example, high-frequency currents, radio waves, microwaves, ultrasonic waves, laser beams, or the like. Preferably, the energy element is a high-frequency electrode that generates high-frequency currents. The energy element 13 in Fig. 2 is a high-frequency electrode (monopolar electrode).

The energy element is connected via a lead wire provided inside the guide tube portion to an energy source at the proximal end of the catheter. As the lead wire, for example, one electrical wire is used for high-frequency currents, a coaxial cable is used for radio waves and microwaves, two electrical wires are used for ultrasonic waves, and a glass fiber is used for laser beams.

The energy element may be provided in a movable manner so as to be protruded from the distal end of the catheter. There is no particular limitation on the movable distance, but it is preferably 3 to 6 mm.

There is no particular limitation on the shape of the high-frequency electrode, but it is preferably a spherical shape, a spatula-like shape, a needle-like shape, a hook-like (hook needle-like) shape, or a fan-like (paddle-like) shape.

According to the catheter of the present invention, for example, the lumen surface can be subjected to incision, cauterization, or hemostasis by coagulation, by pressing a high-frequency electrode in the vicinity of the tip at the distal end against the lumen surface with monitoring using an endoscope. If the catheter of the present invention is provided with an energy element in a movable manner, the operation efficiency for incision, cauterization, or hemostasis by coagulation can be improved. As described above, smoke generated at the time of incision or cauterization can be sucked and removed by the suction means. Although the direction in which a conventional catheter can eject water (irrigation fluid) is limited to the direction of the line of sight of a camera, the catheter of the present invention can eject water (irrigation fluid) not only in the direction of the line of sight of a camera but also in a direction at a right angle with respect to the direction of the line of sight, and, therefore, irrigation of areas around the camera is possible. Moreover, since a plurality of suction openings are provided, the problem of blocking caused by miss-suction of adjacent tissue/organ can be avoided.

### Examples

### (Manufacture of Catheters)

Three catheters were manufactured. These catheters each included a distal end portion that was made of polyether ether ketone (PEEK), a joint that was made of stainless steel (SUS), and a guide tube portion and a proximal end portion that were made of PFA. The vicinity of the tip at the distal end was in the shape of a cylinder having an outer diameter of 2.5 mm and a wall thickness of 0.25 mm, and a plurality of openings each having a diameter of 0.4 mm were arranged in a range within 20 mm in the longitudinal direction from the tip at the distal end. A catheter in which 6 openings per circumference were arranged along the outer circumference for 4 circumferences (4 x 6, 24 openings, Example 1), and catheters in which 8 openings per circumference were arranged along the outer circumference for 3 circumferences (3 x 8, 24 openings) were manufactured. One of the catheters having 3 x 8 openings was manufactured such that 8 openings per circumference were linearly aligned in the longitudinal direction (Example 2), and the other catheter was manufactured such that 8 openings per circumference were sequentially arranged between adjacent circumferences by 15° along the outer circumference (Example 3).

### (Evaluation of Flow Rate Performance)

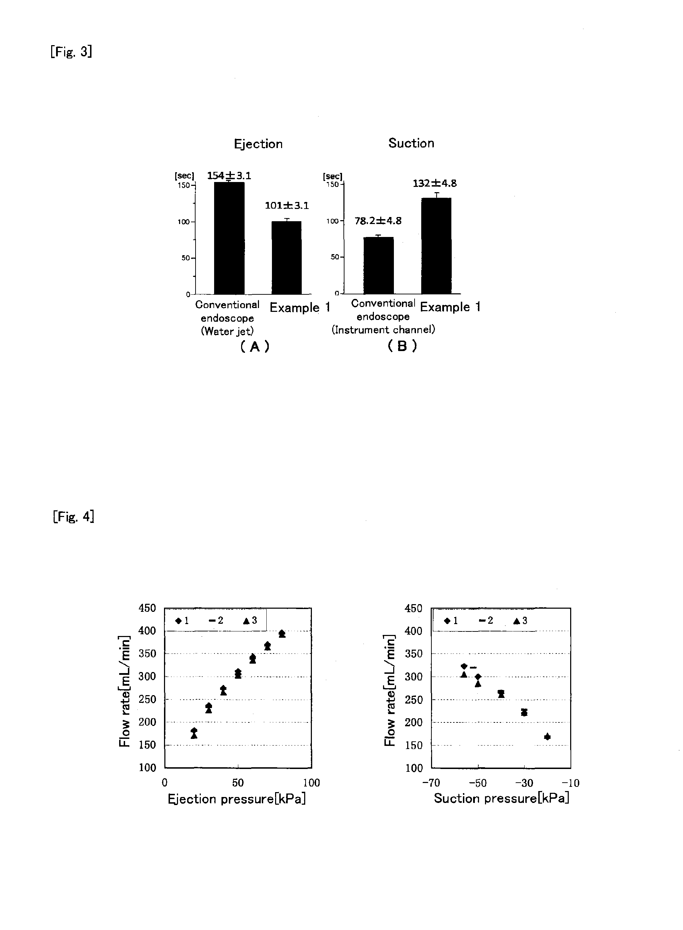

Comparison was performed using the catheter of Example 1 and a conventional endoscope in terms of the flow rate performance. First, 500 mL of physiological saline was supplied at a constant pressure (50 kPa) to the proximal end of the catheter or a water supply opening of a conventional endoscope jet device, the physiological saline was ejected from the openings in the vicinity of the tip at the distal end of the catheter or an ejection opening of the conventional endoscope jet device, and comparison was performed regarding the time required to eject all the physiological saline (ejection test). The distal end of the catheter or an instrument channel (φ 3.2 mm) of the conventional endoscope was immersed in 500 mL of physiological saline, the physiological saline was sucked at a constant pressure (-50 kPa) from the openings in the vicinity of the tip at the distal end of the catheter or the instrument channel of the conventional endoscope, and comparison was performed regarding the time required to suck all the physiological saline (suction test). Figs. 3(A) and 3(B) respectively show the results of the ejection test and the suction test.

As clearly seen from Fig. 3, the catheter of Example 1 can achieve a greater ejection flow rate and a more practical suction flow rate compared with those of the conventional endoscope jet device.

### (Relationship between Pressure and Flow Rate)

Next, in the ejection test and the suction test on the catheters of Examples 1 to 3, the relationship between the ejection pressure and the ejection flow rate and the relationship between the suction pressure and the suction flow rate were checked. The ejection test was performed by supplying 500 mL of physiological saline at a constant pressure to the proximal end of the catheters, causing the physiological saline to be ejected from the openings in the vicinity of the tip at the distal end of the catheters, and measuring the ejection flow rate per minute (mL/min) at each pressure. The suction test was performed by immersing the distal end of the catheters in 500 mL of physiological saline, causing the physiological saline to be sucked at a constant pressure through the openings in the vicinity of the tip at the distal end of the catheters, and measuring the suction flow rate per minute (mL/min) at each pressure. Fig. 4 shows the results of the ejection test and the suction test.

As clearly seen from Fig. 4, the relationship between the ejection pressure and the ejection flow rate and the relationship between the suction pressure and the suction flow rate are expressed as substantially straight lines, and a practical ejection flow rate and a practical suction flow rate can be achieved in the range of 150 to 400 mL/min checked. Note that 1 to 3 in Fig. 4 respectively indicate the catheters of Examples 1 to 3.

### (Safety Test)

Although mucosal internal bleedings on organs or miss-suction of adjacent tissue/organ was observed at the time of suction using conventional suction instruments, no mucosal internal bleedings on organs or miss-suction of adjacent tissue/organ was observed when using the catheters of Examples 1 to 3. Accordingly, it can be assured that the catheters of the present invention have a higher safety level than that of conventional suction instruments.

### Industrial Applicability

The catheter for an endoscope of the present invention can irrigate the lumen surface targeted for procedures, suck the blood, and irrigate and remove cauterized pieces that have adhered to the electrode forceps or the peripheral tissue during incision or cauterization, via a plurality of openings that are arranged in the vicinity of the tip at the distal end of the catheter and that each have a diameter smaller than an inner diameter of the catheter. Furthermore, the catheter for an endoscope of the present invention can have not only the above-described functions but also other functions such as incision, cauterization, hemostasis by coagulation, and smoke evacuation. In this manner, the catheter for an endoscope of the present invention can have multiple functions such as irrigation, suction, incision, cauterization, hemostasis by coagulation, and smoke evacuation. The present invention is a multi-function device obtained by aggregating functions respectively provided in a plurality of conventional instruments in one catheter, and, therefore, the present invention contributes to reducing the burden of a surgeon and shortening the surgical time because instruments do not have to be switched during a surgery.

### Description of Numerals

- 1: catheter
- 2: opening
- 11: catheter
- 12: opening
- 13: energy element

## Claims

1. A catheter for an endoscope, comprising:
an opening in the vicinity of the tip at a distal end thereof,
wherein the opening has a diameter smaller than an inner diameter of the catheter, and
a plurality of said openings are provided.

2. The catheter of claim 1, wherein the endoscope is a flexible endoscope.

3. The catheter of claim 1 or 2,
wherein the vicinity of the tip has a cylindrical shape, and
the number of said openings along an outer circumference of the cylindrical shape is 3 to 12 per circumference.

4. The catheter of any one of claims 1 to 3, further comprising an energy element in the vicinity of the tip.

5. The catheter of any one of claims 1 to 4, wherein the energy element generates at least one selected from the group consisting of a high-frequency current, a radio wave, a microwave, an ultrasonic wave, and a laser beam.
